(19) Europäisches Patentamt
European Patent Office
Office européen des brevets

(11) EP 4 624 521 A1

(12) **EUROPEAN PATENT APPLICATION**
published in accordance with Art. 153(4) EPC

(43) Date of publication:
**01.10.2025 Bulletin 2025/40**

(21) Application number: **23958514.4**

(22) Date of filing: **14.11.2023**

(51) International Patent Classification (IPC):
***C08J 11/28*** *(2006.01)* ***C08J 11/22*** *(2006.01)*
***C08L 75/04*** *(2006.01)*

(52) Cooperative Patent Classification (CPC):
**C08J 11/22; C08J 11/28; C08L 75/04;** Y02W 30/62

(86) International application number:
**PCT/CN2023/131434**

(87) International publication number:
**WO 2025/102228 (22.05.2025 Gazette 2025/21)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Swancor Innovation & Incubation Co., Ltd.**
**Nantou City, Nantou 54066 (TW)**

(72) Inventors:
- **CHEN, Yao-Huang**
  **Nantou City 54066 (TW)**
- **WANG, Meng-Wei**
  **Nantou City 54066 (TW)**

(74) Representative: **Ter Meer Steinmeister & Partner Patentanwälte mbB**
**Nymphenburger Straße 4**
**80335 München (DE)**

# (54) METHOD FOR RECOVERING POLYOL FROM POLYURETHANE WASTE

(57) A method for recovering a polyol from polyurethane waste, comprising the following steps: using a degrading agent to degrade polyurethane waste, and then removing the degrading agent to obtain a degradation product; a treating agent represented by formula (I) reacting with the degradation product to obtain a reaction liquid containing a reaction product and a polyol compound; and distilling the reaction liquid to remove the unreacted treating agent and obtain a polyol mixture. The degradation product contains an amine compound and the polyol compound. The reaction product is formed by the amine compound reacting with the treating agent, and the reaction product is in a liquid state at 20-40°C. The method of the present invention allows the amine compound in the degradation product to react to form a liquid product that does not interfere with subsequent treatment. In addition, the unreacted treating agent can also be removed or recovered by means of distillation to obtain a high-purity polyol mixture.

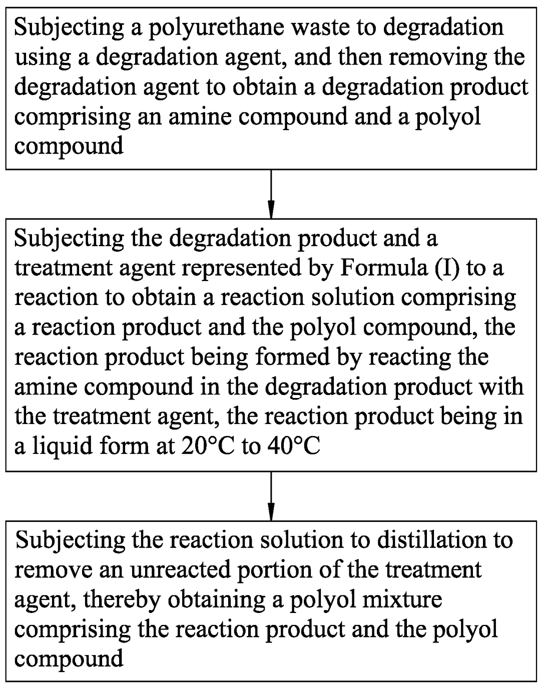


FIG. 1

EP 4 624 521 A1

## Description

### TECHNICAL FIELD

The present disclosure relates to a method for recovering a plastic waste, and more particularly to a method for recovering polyol from a polyurethane waste.

### BACKGROUND TECHNOLOGY

Recycling of a polyurethane waste mostly adopts a chemical degradation method to obtain a degradation product, and then polyol is recovered from the degradation product, or the degradation product serves as a raw material and is further reacted to be made into a product (such as polyurethane). The chemical degradation method includes pyrolysis, hydrolysis, alcoholysis, alkaline hydrolysis, and aminolysis.

Regardless of the chemical degradation method, the degradation product of the polyurethane waste containing both an alcohol compound (such as polyol) and an amine compound can lead to problems in subsequent application of the degradation product. For example, when the degradation product is further made into polyurethane, because a reaction rate of isocyanate and an amine compound is much greater than a reaction rate of isocyanate and polyol, gel is easily formed during the reaction, causing overall reaction to be incomplete, and is also not conducive to the preparation and purification of polyurethane. From the above, it is known that the amine compound contained in the degradation product must be removed or deactivated so as to not affect subsequent applications.

There are patents disclosing the use of a treatment agent to react with an amine compound for its removal. For example, Chinese Invention Patent Publication CN 1290908C mentions a method for treating a polyurethane resin, which includes decomposing a urethane bond of the polyurethane resin to obtain a resin decomposed substance, and then mixing and reacting the resin decomposed substance with an acid anhydride or a compound having one carboxyl group and one hydroxyl group which serves as a treatment agent (acetic acid, lactic acid, salicylic acid, acetic anhydride, formyl chloride, methyl benzoate, butyrolactone and sodium benzoate), so as to obtain a composition to be recycled. The composition to be recycled can be further reacted with an epoxy resin or an isocyanate compound to obtain a recycled resin. In this patent publication, a screw extruder is used to degrade and treat the polyurethane resin, and thus the reactant, degradation agent and treatment agent added during the treatment process of the polyurethane resin cannot be recovered, and may also cause the subsequently made recycled resin to contain a large amount of impurities (such as diluents, fillers, etc.).

However, when the treatment agent is an acid, the acid reacting with the amine in the decomposed substance will produce water and small molecules containing amide group. The water should be removed before proceeding to subsequent applications, causing the treatment process to be time-consuming and complicated. When the small molecules containing an amide group are subsequently made into polyurethane resin, these molecules will be dispersed in the polyurethane resin, causing the mechanical strength of the polyurethane resin to decrease. When the treatment agent is acid anhydride, after the acid anhydride and the amine in the decomposed substance undergo a ring-opening reaction, the polymerization reaction will continue to produce precipitates, which are not conducive to the recovery of polyol or the subsequent preparation of recycled resin. Secondly, when a relatively large amount of acid anhydride is used, the remaining acid anhydride cannot be removed by distillation, which is also not conducive to the recovery of polyol. When the treatment agent is lactone, if the amount of the lactone used is too high, the mechanical strength of the subsequently produced recycled resin will also be affected.

From the description above, it is known that the treatment agent and the amine compound currently used in the industry will generate precipitates and may affect the mechanical strength of the recycled resin produced subsequently. Additionally, the unreacted treatment agent cannot be effectively removed by distillation process. Therefore, there is a still a need for continuation of research and development of treatment agents in the industry.

### SUMMARY

Therefore, the present disclosure aims to provide a method for recovering polyol from a polyurethane waste. The method effectively allows an amine compound in the degradation product to react so as to generate products in a liquid form which do not affect subsequent treatment, and also allows unreacted treatment agent to be removed or recovered by distillation, thereby obtaining a polyol mixture with high purity.

Accordingly, in the present disclosure, the method for recovering polyol from a polyurethane waste includes the steps of:

subjecting the polyurethane waste to degradation using a degradation agent, and then removing the degradation agent to obtain a degradation product comprising an amine compound and a polyol compound;

subjecting the degradation product and a treatment agent represented by Formula (I) to a reaction to obtain a reaction solution including a reaction product and the polyol compound, the reaction product being formed by reacting the amine compound in the degradation product with the treatment agent, the reaction product being in a liquid form at 20°C to 40°C; and

subjecting the reaction solution to distillation to remove an unreacted portion of treatment agent, thereby obtaining a polyol mixture including the reaction product and the polyol compound;

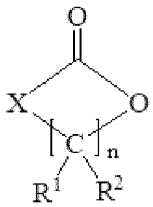

Formula (I)

wherein in Formula (I),

X is N-R, R is hydrogen or a straight chain alkyl group;
n is an integer ranging from 2 to 4;
$R^1$ and $R^2$ each represents hydrogen, a straight chain alkyl group or a branched chain alkyl group;
$R^1$ on different carbon atoms is the same or different; and
$R^2$ on different carbon atoms is the same or different.

In certain embodiments of the method, X is O. In certain preferred embodiments of the present disclosure, the treatment agent is ethylene carbonate, propylene carbonate, 1,2-butanediol carbonate, or combinations thereof.

In certain embodiments of the present disclosure, X is N-R. In certain preferred embodiments of the present disclosure, the treatment agent is 2-oxazolidinone (R is H), 4-methyl-2-oxazolidinone (R is H), 3-methyl-2-oxazolidinone (R is $CH_3$), or combinations thereof.

In certain embodiments of present disclosure, an amount of the treatment agent used is calculated by the following equation:

$$\text{Amount of the treatment agent used (g)} = \text{weight of the degradation product (g)} \times (\text{an amine value of the degradation product}/1000) \times \frac{1}{56.1} \times \text{a molecular weight of the treatment agent} \times N$$

; where N is a value ranging from 0.1 to 5.

In certain embodiments of the present disclosure, the amine compound contained in the degradation product is an aliphatic amine compound, an aromatic amine compound, an alcoholamine compound, or combinations thereof.

In certain embodiments of the present disclosure, the degradation product and the treatment agent is subjected to the reaction at a temperature ranging from 50°C to 150°C.

In certain embodiments of the present disclosure, the reaction solution is subjected to distillation at a temperature ranging from 70°C to 160°C and a pressure ranging from 0.001 mbar to 100 mbar.

In certain embodiments of the present disclosure, the degradation agent is an amine compound, an alcohol compound, an alcoholamine compound, or combinations thereof.

In certain embodiments of the present disclosure, the alcohol compound is a polyol compound, and the polyol compound is not removed during removal of the degradation agent.

In certain embodiments of the present disclosure, the reaction product is ureido-polyol, urethane polyol, and a combination thereof.

In certain embodiments of the present disclosure, the polyol mixture has an infrared absorption peak at a wavenumber ranging from 1730 $cm^{-1}$ to 1745 $cm^{-1}$.

In certain embodiments of the present disclosure, the polyol mixture is ureido-polyol, urethane polyol, polyoxypropylene polyol, or combinations thereof.

The advantageous effects of the present disclosure are, by subjecting the treatment agent represented by Formula (I) and the amine compound in the degradation product to the reaction, in addition to the absence of serious gelation occurring during the reaction, the reaction product thus obtained is in a liquid form. Therefore, during subsequent treatment, the reaction solution does not need to be filtered to remove the reaction product that is formed by the reaction of the treatment compound and the amine compound, and the unreacted portion of the treatment agent can also be removed or recovered by distillation, so that the polyol mixture with a high purity can finally be obtained.

## BRIEF DESCRIPTION OF THE DRAWINGS

Other features and advantages of the present disclosure will become apparent in the following detailed description of the embodiment(s) with reference to the accompanying drawings.

FIG 1 is a flow diagram illustrating the steps of a method according to the present disclosure.
FIG. 2 is an infrared absorption spectrum of a degradation product obtained according to one embodiment of the present disclosure.
FIG. 3 is an infrared absorption spectrum of a polyol mixture obtained according to one embodiment of the present disclosure.

## DETAILED DESCRIPTION

The method for recovering polyol from a polyurethane waste of the present disclosure includes the steps of: subjecting the polyurethane waste to degradation using a degradation agent, and then removing the degradation agent to obtain a degradation product including an amine compound and a polyol compound; subjecting the degradation product and a treatment agent represented by Formula (I) to a reaction to obtain a reaction solution including a reaction product and the polyol compound, the reaction product being formed by reacting the amine compound in the degradation product with the treatment agent, the reaction product being in a liquid form at 20°C to 40°C; and subjecting the reaction solution to distillation to remove an unreacted portion of the treatment agent, thereby obtaining a polyol mixture including the reaction product and the polyol compound;

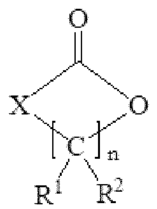 Formula (I).

According to the present disclosure, the polyurethane waste may refer to any polyurethane material that is to be discarded, such as polyurethane foam, polyurethane leather, polyurethane soles, etc. The polyurethane material may be prepared by subjecting one or several types of isocyanate molecules or one or several types of reactive molecules to a polymerization reaction. The isocyanate molecules may be roughly divided into two types, i.e., aromatic diisocyanate or derivatives thereof, and aliphatic diisocyanate or derivatives thereof. In certain embodiments of the present disclosure, examples of the aromatic diisocyanate include, but are not limited to, toluene diisocyanate (TDI), methylene diphenyl diisocyanate (MDI), naphthalene diisocyanate (NDI), p-phenylene diisocyanate (PPDI), xylylene diisocyanate (XDI), dimethyl biphenylene diisocyanate (TODI), and dimethyl methylene diphenyl diisocyanate (DMMDI). In certain embodiments of the method, the aromatic diisocyanate is toluene diisocyanate (TDI), methylene diphenyl diisocyanate (MDI), or p-phenylene diisocyanate (PPDI). In certain embodiments of the present disclosure, examples of the derivatives of aromatic diisocyanate include, but are not limited to, toluene diisocyanate dimer (TDI-dimer), toluene diisocyanate trimer (TDI-trimer), and poly(methylene diphenyl diisocyanate) (PMDI). Examples of the aliphatic diisocyanate include, but are not limited to, isophorone diisocyanate (IPDI), hexamethylene diisocyanate (HDI), dicyclohexylmethane diisocyanate ($H_{12}MDI$), 1,4-cyclohexyl diisocyanate (CHDI), trimethyl-1,6-hexamethylene diisocyanate (TMHDI), and methylcyclohexyl diisocyanate (HTDI). In certain embodiments of the method, the aliphatic diisocyanate is isophorone diisocyanate (IPDI), hexamethylene diisocyanate (HDI), dicyclohexylmethane diisocyanate ($H_{12}MDI$), or 1,4-cyclohexyl diisocyanate (CHDI). In certain embodiments of the present disclosure, examples of the derivatives of aliphatic diisocyanate include, but are not limited to, hexamethylene diisocyanate dimer (HDI-dimer), hexamethylene diisocyanate trimer (HDI-trimer), hexamethylene diisocyanate biuret (HDI Biuret), and isophorone diisocyanate trimer (IPDI-trimer).

Examples of the reactive molecules include, but are not limited to, polyester polyol, polyether polyol, water, diol, polyol, alkamine, and diamine. Examples of the polyester polyol include, but are not limited to: (1) adipic acid polyester polyol, which is obtained by subjecting adipic acid and one or several types of diols (such as ethylene glycol, propylene glycol, butylene glycol, diethylene glycol, etc.) to a polycondensation reaction, which has a hydroxyl value ranging from 25 to 200 mgKOH/g and an average molecular weight ranging from 500 to 5000; (2) aromatic polyester polyol, which is prepared by subjecting one or several types of aromatic dianhydrides (such as phthalic anhydride (PA), terephthalic acid (PTA), isophthalic acid (IPA)) and one or several types of diols (such as ethylene glycol, propylene glycol, butylene glycol, diethylene glycol, etc.) to a polycondensation reaction and has an average molecular weight ranging from 200 to 2000; (3) polycaprolactone diol, which is prepared by subjecting ε-caprolactone and one or several types of diols (such as butylene

glycol, neopentyl glycol, hexanediol, ethylene glycol, diethylene glycol, etc.) to a ring-opening polymerization reaction and has an average molecular weight ranging from 300 to 4000; and (4) polycarbonate diol, which is prepared by subjecting one or several types of diols (such as 1,6-hexanediol, 1,4-butanediol, 1,4-cyclohexanedimethanol, neopentyl glycol, 1,5-pentanediol, 3-methylpentanediol, etc.) and one or several types of carbonate esters (such as dimethyl carbonate, diethyl carbonate, dipropyl carbonate, diphenyl carbonate, ethylene carbonate, propylene carbonate, etc.) to a transesterification reaction and has an average molecular weight ranging from 500 to 3000. Examples of the polyether polyol include, but are not limited to: (1) polyoxypropylene polyol, which is prepared by subjecting one or several types of hydroxyl-containing molecules (such as 1,6-hexanediol, 1,4-butanediol, propylene glycol, ethylene glycol, neopentyl glycol, 1,5-pentanediol, 3-methylpentanediol, diethylene glycol, dipropylene glycol, triethylene glycol, glycerol, trimethylolpropane, ethanolamine, diethanolamine, triethanolamine, etc.) and propylene oxide to a polymerization reaction and has an average molecular weight ranging from 200 to 8000; (2) polyoxyethylene polyol, which is prepared by subjecting ethylene glycol, diethylene glycol or a combination thereof and ethylene oxide to a polymerization reaction and has an average molecular weight ranging from 200 to 20000; (3) polytetrahydrofuran polyol, which is prepared by subjecting tetrahydrofuran to a ring-opening polymerization and has an average molecular weight ranging from 1000 to 3000; (4) polymer polyol, which is a styrene-acrylonitrile graft polymer polyol based on propylene oxide (PO)-ethylene oxide (EO) copolyether triol and has a hydroxyl value ranging from 15 to 75; and (5) polytrimethylene ether polyol, which is prepared by subjecting 1,3-propylene glycol to polymerization and has an average molecular weight ranging from 600 to 2500. Examples of the diol include, but are not limited to, ethylene glycol, 1,4-butanediol, diethylene glycol, triethylene glycol, 1,2-propylene glycol, neopentyl glycol, methylpropylene glycol, 1,6-hexanediol, 1,3-propylene glycol, dipropylene glycol, tripropylene glycol, butylethylpropylene glycol, diethyl pentanediol, 3-methyl-1,5-pentanediol, 1,3-butanediol, 1,2-butanediol, 2,3-butanediol, trimethyl pentanediol, cyclohexanediol, 1,4-dihydroxymethylcyclohexane, etc. Examples of the polyol may include, but not limited to, trimethylolpropane, glycerol, trimethyloethane, 1,2,6-hexanetriol, tris(hydroxyethyl) isocyanurate, pentaerythritol, xylitol, sorbitol, etc. Examples of the alcoholamine may include, but not limited to, triethanolamine, diethanolamine, triisopropanolamine, methyldiethanolamine, bis(hydroxyisopropyl) aniline, bis(hydroxyisopropyl) p-toluidine, dihydroxyethylaniline, dihydroxyethyl-p-toluidine, and dihydroxyethyl-m-toluidine. Examples of the diamine include, but are not limited to, 3,3'-dichloro-4,4'-diphenylmethanediamine, 3,5-dimethylthiotoluenediamine, 3,5-diethyltoluenediamine, 4,4'-methylene bis(3-chloro-2,6-diethylaniline), 4,4'-methylene bis(2,6-diethylaniline), 4,4'-methylene bis(2,6-diisopropylaniline), 4,4'-methylene bis(2-isopropyl-6-methylaniline), 4,4'-methylene bis(2-isopropyl-6-diethylaniline), 4,4'-methylene bis(2-ethylaniline), toluenediamine, 4,4'-diaminodiphenylmethane, isophorone diamine, diaminodicyclohexylmethane, trimethylhexamethylenediamine, and 4,4'-methylenebis(2-methylcyclohexylamine).

In the present disclosure, the degradation of the polyurethane waste may be conducted in accordance to a chemical degradation method well known to those skilled in the art, and may be conducted for one time or multiple times using any known degradation agent to break the urethane or urea bonds of polyurethane and further degrading the same into polyols or amines, etc. In certain embodiments of the present disclosure, the degradation agent is an amine compound, an alcohol compound (such as a polyol compound), an alcoholamine compound, or combinations thereof. In an embodiment, the degradation agent is an alcoholamine compound and an alcohol compound. In another embodiment, the alcohol compound is a polyol compound. It should be noted that, when the degradation agent is a polyol compound or contains a polyol compound, after the degradation agent is used in the degradation reaction, the polyol compound is not removed, that is, in the step of removing the degradation agent, only other compounds other than the polyol compound are removed.

In certain embodiments of the present disclosure, the way the degradation agent is removed is related to the form of the degradation product, and the form of the degradation product depends on the molecular weight of the polyol contained in the degradation product. When the molecular weight of the polyol contained in the degradation product is less than 1000, the degradation product is in a liquid form. When the molecular weight of the polyol contained in the degradation product ranges from 1000 to 3000, the degradation product is in a paste form. When the molecular weight of the polyol contained in the degradation product is greater than 3000, the degradation product is in a non-fluid fat form. The way the degradation agent is removed is different, depending on the different forms as mentioned above. The degradation agent and the degradation product in the liquid or paste form will separate into layers, and sampling can be conducted according to the layers. The degradation agent can be directly poured out from the degradation product that is in the non-fluid fat form.

The degradation product obtained after degradation of the polyurethane waste contains one or several types of the amine compounds and one or several types of the polyol compounds. In certain embodiments of the present disclosure, amine compound is an aliphatic amine compound, an aromatic amine compound, an alcoholamine compound, or combinations thereof.

The treatment agent used in the present disclosure is represented by the following Formula (I):

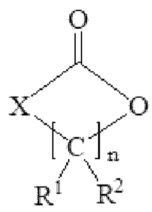

Formula (I).

According to the present disclosure, in Formula (I), X is O or N-R, R is hydrogen or a straight chain alkyl group; n is an integer ranging from 2 to 4; $R^1$ and $R^2$ each represents hydrogen, a straight chain alkyl group or a branched chain alkyl group; $R^1$ on different carbon atoms is the same or different; and $R^2$ on different carbon atoms is the same or different.

In certain embodiments of the present disclosure, $R^1$ and $R^2$ each is a straight chain alkyl group or a branched chain alkyl group, and a carbon number thereof ranges from 1 to 10. In certain embodiments of the present disclosure, the carbon number of the straight chain alkyl group or the branched chain alkyl group ranges from 1 to 5. In a specific embodiment of the present disclosure, the carbon number of the straight chain alkyl group or the branched chain alkyl group ranges from 1 to 3.

In certain embodiments of the present disclosure, the treatment agent is represented by the following Formula (I-1):

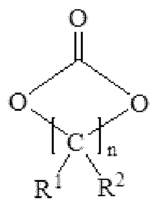

Formula (I-1).

Wherein, in Formula (I-1), X is O, and the definitions for n, $R^1$ and $R^2$ are the same as in Formula (I).

In certain embodiments of the present disclosure, the treatment agent is ethylene carbonate, propylene carbonate, 1,2-butanediol carbonate, or combinations thereof.

Ethylene carbonate

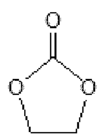

Propylene carbonate

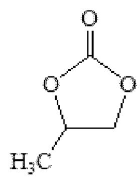

1,2-butanediol carbonate

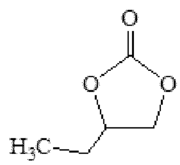

In certain embodiments of the present disclosure, X is N-R, and the treatment agent is represented by the following Formula (I-2):

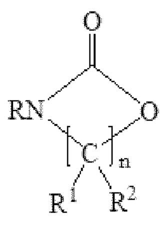

Formula (I-2).

According to the present disclosure, in Formula (I-2), the definitions for n, $R^1$ and $R^2$ are the same as in Formula (I). In certain embodiments of the present disclosure, R is hydrogen or methyl group.

In certain embodiments of the present disclosure, the treatment agent is 2-oxazolidinone, 4-methyl-2-oxazolidinone, 3-methyl-2-oxazolidinone, or combinations thereof (shown below).

2-oxazolidinone

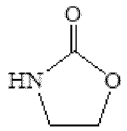

4-methyl-2-oxazolidinone

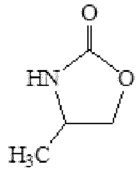

3-methyl-2-oxazolidinone

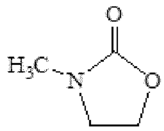

In a specific embodiment, the treatment agent is ethylene carbonate, propylene carbonate, 1,2-butanediol carbonate, 2-oxazolidinone, 4-methyl-2-oxazolidinone, 3-methyl-2-oxazolidinone, or combinations thereof.

The temperature and pressure of the reaction of the degradation product and the treatment agent may be adjusted according to practical requirements. In certain embodiments of the present disclosure, the degradation product and the treatment agent is subjected to the reaction at a temperature ranging from 50°C to 150°C. In certain embodiments of the present disclosure, the temperature ranges from 90°C to 150°C. In a specific embodiment, the temperature is 120°C. In another specific embodiment, the temperature is 90°C. In yet another specific embodiment, the temperature is 130°C.

The amount of the treatment agent used is mainly adjusted in accordance to the amine compound in the degradation product. In certain embodiments of the present disclosure, the amount of the treatment agent used is calculated by the following equation:

$$\text{Amount of the treatment agent used (g)} = \text{weight of the degradation product (g)} \times (\text{the amine value of the degradation product}/1000) \times \frac{1}{56.1} \times \text{the molecular weight of the treatment agent} \times N$$

; where N is a value ranging from 0.1 to 5. In certain embodiments of the present disclosure, N is a value ranging from 0.3 to 1.5.

In the reaction of the treatment agent and the degradation product, the treatment agent and the amine compound in the degradation product are subjected to a ring-opening reaction to form a reaction product. The reaction product is in a liquid form at room temperature (20°C to 40°C), indicating that precipitate will not be formed after the reaction of the treatment agent and the degradation product, and that the purity of the polyol mixture and subsequent applications will not be affected. In certain embodiments of the present disclosure, the reaction product is ureido-polyol, urethane polyol, and a combination thereof.

The temperature and pressure in the step of the reaction solution being subjected to distillation may be adjusted according to the treatment agent used. In certain embodiments of the present disclosure, the reaction solution is subjected to distillation at a temperature ranging from 70°C to 160°C and a pressure ranging from 0.001 mbar to 100 mbar. In certain embodiments of the present disclosure, the reaction solution is subjected to distillation at a temperature ranging from 75°C to 150°C and a pressure ranging from 0.01 mbar to 20 mbar.

The reaction solution may be optionally subjected to filtration before distillation to remove insoluble matter, and further improving the purity of the polyol mixture.

The polyol mixture thus obtained contains the reaction product and the polyol compound. In certain embodiments of the present disclosure, the thus obtained polyol mixture includes ureido-polyol, urethane polyol, polyoxypropylene polyol, or combinations thereof. It should be noted that, when the composition of the polyurethane waste includes alkyl diol (such as ethylene glycol, 1,4-butanediol, etc.), the polyol compound thus obtained after degradation of the polyurethane waste contains alkyl diol, and the polyol mixture finally obtained also contains alkyl diol.

In certain embodiments of the present disclosure, the polyol mixture thus obtained has an infrared absorption peak at a wavenumber ranging from 1730 $cm^{-1}$ to 1745 $cm^{-1}$. In a specific embodiment of the present disclosure, the polyol mixture has an infrared absorption peak at 1736 $cm^{-1}$.

The polyol mixture obtained by the method of the present disclosure may be subsequently used to prepare thermosetting polyurethane, etc.

**EXAMPLES**

The present disclosure will be described by way of the following examples. However, it should be understood that the following examples are intended solely for the purpose of illustration and should not be construed as limiting the present disclosure in practice.

## Test Method

The amine value, hydroxyl value, and viscosity of the examples below were determined according to the following tests:

1. Amine value (mgKOH/g) was determined according to ISO 25761:2014 standard test method.
2. Hydroxyl value (mgKOH/g) was determined according to ASTM D4274 standard test method.
3. Viscosity (cP) was determined according to ISO 3219 standard test method, and the test was conducted at 25°C.

## Example 1 Preparation of degradation product

1500 g of soft PU foam (containing 60 wt% of polyol) was mixed with 3000 g of a first degradation agent, and then subjected to a first degradation reaction at 135°C to obtain a solid-liquid layered degradation reaction solution (containing fatty product and reaction mixture solution). The first degradation agent includes an alcoholamine compound, which refers to a multifunctional compound having both hydroxyl (-OH) and amine ($-NH_2$ and -NHR) functional groups, such as diethanolamine, bis(2-hydroxyisopropyl)aniline, bis(2-hydroxyisopropyl)-p-toluidine, dihydroxyethylaniline, dihydroxyethyl-p-toluidine, dihydroxyethyl-m-toluidine, but are not limited thereto. Next, about 3000 g of the reaction mixture solution after the first degradation reaction was removed (including the first degradation agent, urea diol and toluene diamine), and 1500 of the fatty product was mixed with 150 g of a second degradation agent (glycerol) and subjected to a second degradation reaction at 150°C, thereby obtaining a degradation product.

## Property Evaluation

1. The degradation product was subjected to analysis using an infrared spectrometer (IR, Manufacturer: PerkinElmer, Model no.: Spectrum 100). The result is shown in FIG. 2. As shown in FIG. 2, the degradation product includes an amine compound (toluene diamine) and a polyol compound (polyoxypropylene polyol).
2. The amine value of the degradation product is 100 mgKOH/g, and the estimated output of polyol contained in the degradation product is 800 g.

## Example 2 Use of ethylene carbonate as treatment agent for recovering polyol

100 g of the degradation product of Example 1 was placed in a reactor. Then, 31.4 g (N=2) of ethylene carbonate as a treatment agent was slowly added into the reactor at 25°C. The degradation product was mixed with ethylene carbonate by stirring for 0.5 hours to obtain a solution mixture. Then, the solution mixture was continuously stirred to react at 120°C. During the reaction, the signal change of ethylene carbonate in the solution mixture was continuously monitored using the IR analysis until the signal remained unchanged. That is, the reaction of the solution mixture was terminated, and a reaction solution was obtained.

By observing the reaction solution with naked eye, it was found that the reaction solution was in a liquid form and did not contain precipitate, demonstrating that the reaction product (which was formed by reacting the ethylene carbonate and the amine compound) contained in the reaction solution was also in a liquid form, and did not precipitate in a solid form.

The reaction solution was distilled at 150°C and 20 mbar for 2 hours to remove unreacted ethylene carbonate. Finally, 110.2 g of a polyol mixture (containing urethane polyol and polyoxypropylene polyol) was obtained.

## Property Evaluation

The polyol mixture of Example 2 has a hydroxyl value of 95 mgKOH/g, a viscosity of 1200 cP, and a recovery yield of 110%.

## Example 3 Use of propylene carbonate as treatment agent for recovering polyol

100 g of the degradation product of Example 1 was placed in a reactor. Then, 27.2 g (N=1.5) of propylene carbonate as a treatment agent was slowly added into the reactor at 25°C. The degradation product was mixed with propylene carbonate by stirring for 0.5 hours to obtain a solution mixture. Then, the solution mixture was continuously stirred to react at 120°C. During the reaction, the signal change of propylene carbonate in the solution mixture was continuously monitored using the IR analysis until the signal remained unchanged. That is, the reaction of the solution mixture was terminated, and a reaction solution was obtained.

The reaction solution was distilled at 150°C and 20 mbar for 2 hours to remove unreacted propylene carbonate. Finally, 119.5 g of a polyol mixture (containing urethane polyol and polyoxypropylene polyol) was obtained.

**Property Evaluation**

1. The degradation product was subjected to analysis using infrared spectrometer. The result is shown in FIG. 3. In comparison with FIG. 2, a specific infrared absorption peak can be observed at a wavenumber of 1736 $cm^{-1}$ in FIG. 3.
2. The polyol mixture of Example 3 has a hydroxyl value of 93 mgKOH/g, a viscosity of 1100 cP, and a recovery yield of 120%.

**Example 4 Use of 1,2-butanediol carbonate as treatment agent for recovering polyol**

100 g of the degradation product of Example 1 was placed in a reactor. Then, 20.6 g (N=1) of 1,2-butanediol carbonate as a treatment agent was slowly added into the reactor at 25°C. The degradation product was mixed with 1,2-butanediol carbonate by stirring for 0.5 hours to obtain a solution mixture. Then, the solution mixture was continuously stirred to react at 120°C. During the reaction, the signal change of 1,2-butanediol carbonate in the solution mixture was continuously monitored using the IR analysis until the signal remained unchanged. That is, the reaction of the solution mixture was terminated, and a reaction solution was obtained.

The reaction solution was distilled at 150°C and 20 mbar for 2 hours to remove unreacted 1,2-butanediol carbonate. Finally, 105.5 g of a polyol mixture (containing urethane polyol and polyoxypropylene polyol) was obtained.

**Property Evaluation**

The polyol mixture of Example 4 has a hydroxyl value of 100 mgKOH/g, a viscosity of 1020 cP, and a recovery yield of 105%.

**Example 5 Use of 2-oxazolidinone as treatment agent for recovering polyol**

100.0 g of the degradation product of Example 1 was placed in a reactor. Then, 47.0 g (N=3) of 2-oxazolidinone as a treatment agent was slowly added into the reactor at 25°C. The degradation product was mixed with 2-oxazolidinone by stirring for 0.5 hours to obtain a solution mixture. Then, the solution mixture was continuously stirred to react at 120°C. During the reaction, the signal change of 2-oxazolidinone in the solution mixture was continuously monitored using the IR analysis until the signal remained unchanged. That is, the reaction of the solution mixture was terminated, and a reaction solution was obtained.

The reaction solution was distilled at 120°C and 20 mbar for 2 hours to remove unreacted 2-oxazolidinone. Finally, 110.5 g of a polyol mixture (containing ureido-polyol and polyoxypropylene polyol) was obtained.

**Property Evaluation**

The polyol mixture of Example 5 has a hydroxyl value of 96 mgKOH/g, a viscosity of 1150 cP, and a recovery yield of 110.5%.

**Example 6 Use of 4-methyl-2-oxazolidinone as treatment agent for recovering polyol**

100.0 g of the degradation product of Example 1 was placed in a reactor. Then, 27.2 g (N=1.5) of 4-methyl-2-oxazolidinone as a treatment agent was slowly added into the reactor at 25°C. The degradation product was mixed with 4-methyl-2-oxazolidinone by stirring for 0.5 hours to obtain a solution mixture. Then, the solution mixture was continuously stirred to react at 90°C. During the reaction, the signal change of 4-methyl-2-oxazolidinone in the solution mixture was continuously monitored using the IR analysis until the signal remained unchanged. That is, the reaction of the solution mixture was terminated, and a reaction solution was obtained.

The reaction solution was distilled at 75°C and 20 mbar for 2 hours to remove unreacted 4-methyl-2-oxazolidinone. Finally, 108.0 g of a polyol mixture (containing ureido-polyol and polyoxypropylene polyol) was obtained.

**Property Evaluation**

The polyol mixture of Example 6 has a hydroxyl value of 97 mgKOH/g, a viscosity of 1050 cP, and a recovery yield

of 108%.

**Example 7 Use of 3-methyl-2-oxazolidinone as treatment agent for recovering polyol**

100.0 g of the degradation product of Example 1 was placed in a reactor. Then, 36.0 g (N=2) of 3-methyl-2-oxazolidinone as a treatment agent was slowly added into the reactor at 25°C. The degradation product was mixed with 3-methyl-2-oxazolidinone by stirring for 0.5 hours to obtain a solution mixture. Then, the solution mixture was continuously stirred to react at 130°C. During the reaction, the signal change of 3-methyl-2-oxazolidinone in the solution mixture was continuously monitored using the IR analysis until the signal remained unchanged. That is, the reaction of the solution mixture was terminated, and a reaction solution was obtained.

The reaction solution was distilled at 150°C and 20 mbar for 2 hours to remove unreacted 3-methyl-2-oxazolidinone. Finally, 102.2 g of a polyol mixture (containing ureido-polyol and polyoxypropylene polyol) was obtained.

**Property Evaluation**

The polyol mixture of Example 7 has a hydroxyl value of 95 mgKOH/g, a viscosity of 1000 cP, and a recovery yield of 102.2%.

The examples above prove that, in the method for recovering polyol of the present disclosure, by subjecting the treatment agent represented by Formula (I) to react with the amine compound in the degradation product, in addition to the reaction product being in a liquid form and will not affect subsequent application, the reaction solution does not need to be filtered to remove the reaction product that is formed by reacting the amine compound and the treatment agent during the subsequent step, and for the selected treatment agent, the unreacted portion of the treatment agent can be removed or recovered by vacuum distillation, so as to finally obtain the polyol mixture with high purity. In comparison with a conventional method for removing treatment agent by washing with water, the method of the present disclosure is capable of removing or recovering the unreacted portion of the treatment agent without consuming a large amount of water.

In the description above, for the purposes of explanation, numerous specific details have been set forth in order to provide a thorough understanding of the embodiment(s). It will be apparent, however, to one skilled in the art, that one or more other embodiments may be practiced without some of these specific details. It should also be appreciated that reference throughout this specification to "one embodiment," "an embodiment," an embodiment with an indication of an ordinal number and so forth means that a particular feature, structure, or characteristic may be included in the practice of the disclosure. It should be further appreciated that in the description, various features are sometimes grouped together in a single embodiment, figure, or description thereof for the purpose of streamlining the disclosure and aiding in the understanding of various inventive aspects; such does not mean that every one of these features needs to be practiced with the presence of all the other features. In other words, in any described embodiment, when implementation of one or more features or specific details does not affect implementation of another one or more features or specific details, said one or more features may be singled out and practiced alone without said another one or more features or specific details. It should be further noted that one or more features or specific details from one embodiment may be practiced together with one or more features or specific details from another embodiment, where appropriate, in the practice of the disclosure.

While the disclosure has been described in connection with what is (are) considered the exemplary embodiment(s), it is understood that this disclosure is not limited to the disclosed embodiment(s) but is intended to cover various arrangements included within the spirit and scope of the broadest interpretation so as to encompass all such modifications and equivalent arrangements.

**Claims**

**1.** A method for recovering polyol from a polyurethane waste, **characterized in** comprising the steps of:

subjecting the polyurethane waste to degradation using a degradation agent, and then removing the degradation agent to obtain a degradation product comprising an amine compound and a polyol compound;
subjecting the degradation product and a treatment agent represented by Formula (I) to a reaction to obtain a reaction solution comprising a reaction product and the polyol compound, the reaction product being formed by reacting the amine compound in the degradation product with the treatment agent, the reaction product being in a liquid form at 20°C to 40°C; and
subjecting the reaction solution to distillation to remove an unreacted portion of the treatment agent, thereby obtaining a polyol mixture comprising the reaction product and the polyol compound;

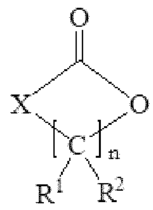

Formula (I)

wherein in Formula (I),

X is N-R, R is hydrogen or a straight chain alkyl group;
n is an integer ranging from 2 to 4;
$R^1$ and $R^2$ each represents hydrogen, a straight chain alkyl group or a branched chain alkyl group;
$R^1$ on different carbon atoms is the same or different; and
$R^2$ on different carbon atoms is the same or different.

2. The method as claimed in claim 1, **characterized in that**, X is O.

3. The method as claimed in claim 2, **characterized in that**, the treatment agent is ethylene carbonate, propylene carbonate, 1,2-butanediol carbonate, or combinations thereof.

4. The method as claimed in claim 1, **characterized in that**, X is N-R.

5. The method as claimed in claim 4, **characterized in that**, the treatment agent is 2-oxazolidinone, 4-methyl-2-oxazolidinone, 3-methyl-2-oxazolidinone, or combinations thereof. The method as claimed in claim 1, wherein the treatment agent is 2-oxazolidinone, 4-methyl-2-oxazolidinone, 3-methyl-2-oxazolidinone or combinations thereof.

6. The method as claimed in claim 1, **characterized in that**, an amount of the treatment agent used is calculated by the following equation:

$$\text{Amount of the treatment agent used (g)} = \text{weight of the degradation product (g)} \times (\text{an amine value of the degradation product}/1000) \times \frac{1}{56.1} \times \text{a molecular weight of the treatment agent} \times N;$$

where N is a value ranging from 0.1 to 5.

7. The method as claimed in claim 1, **characterized in that**, the amine compound of the degradation product is an aliphatic amine compound, an aromatic amine compound, an alcoholamine compound, or combinations thereof.

8. The method as claimed in claim 1, **characterized in that**, the degradation product and the treatment agent is subjected to the reaction at a temperature ranging from 50°C to 150°C.

9. The method as claimed in claim 1, **characterized in that**, the reaction solution is subjected to distillation at a temperature ranging from 70°C to 160°C and a pressure ranging from 0.001 mbar to 100 mbar.

10. The method as claimed in claim 1, **characterized in that**, the degradation agent is an amine compound, an alcohol compound, an alcoholamine compound, or combinations thereof.

11. The method as claimed in claim 10, **characterized in that**, the alcohol compound is a polyol compound, and the polyol compound is not removed during removal of the degradation agent.

12. The method as claimed in claim 1, **characterized in that**, the reaction product is ureido-polyol, urethane polyol, and a combination thereof.

13. The method as claimed in claim 1, **characterized in that**, the polyol mixture has an infrared absorption peak at a wavenumber ranging from 1730 $cm^{-1}$ to 1745 $cm^{-1}$.

**14.** The method as claimed in claim 1, **characterized in that**, the polyol mixture is ureido-polyol, urethane polyol, polyoxypropylene polyol, or combinations thereof.

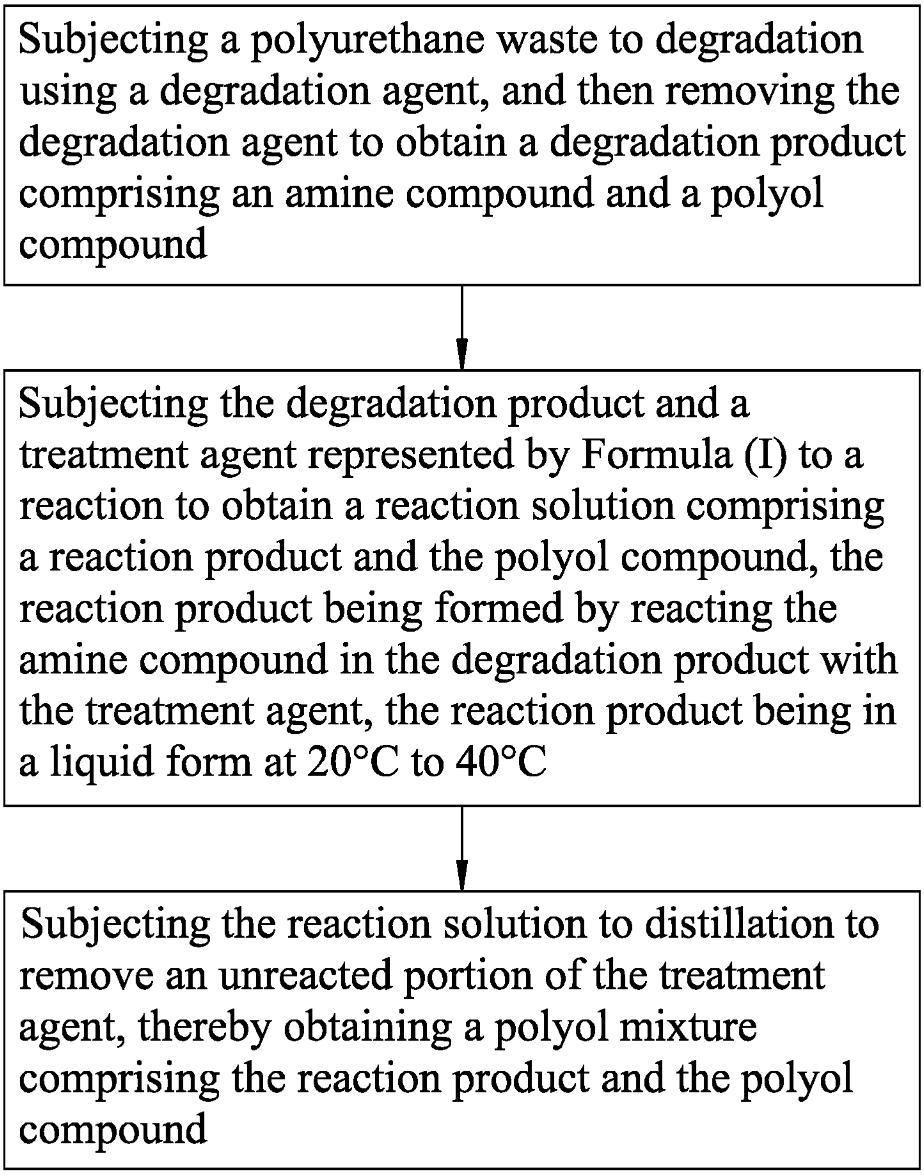
Subjecting a polyurethane waste to degradation using a degradation agent, and then removing the degradation agent to obtain a degradation product comprising an amine compound and a polyol compound
Subjecting the degradation product and a treatment agent represented by Formula (I) to a reaction to obtain a reaction solution comprising a reaction product and the polyol compound, the reaction product being formed by reacting the amine compound in the degradation product with the treatment agent, the reaction product being in a liquid form at 20°C to 40°C
Subjecting the reaction solution to distillation to remove an unreacted portion of the treatment agent, thereby obtaining a polyol mixture comprising the reaction product and the polyol compound

FIG. 1

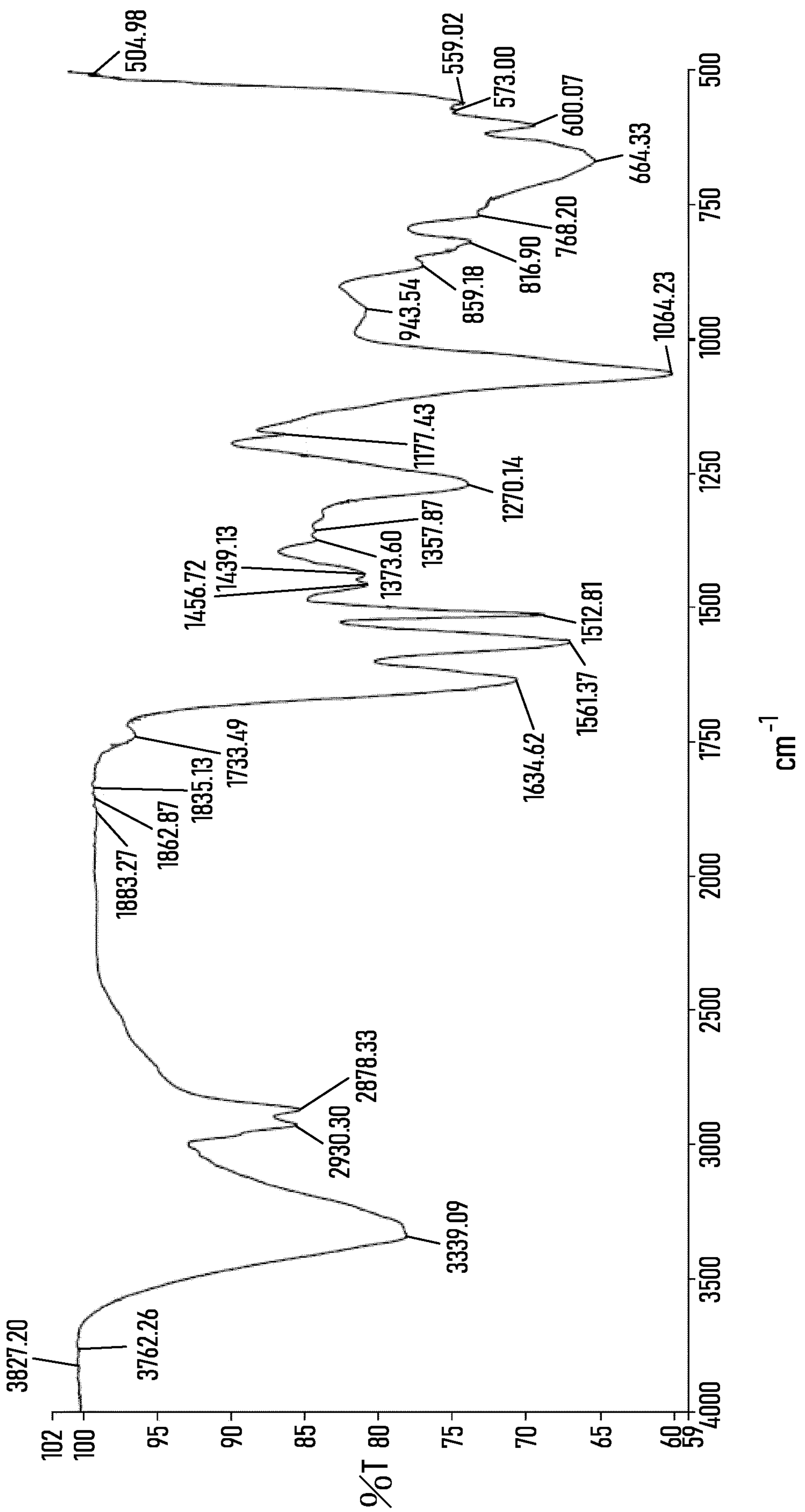
%T
cm-1
102
100
95
90
85
80
75
70
65
60
59
4000
3500
3000
2500
2000
1750
1500
1250
1000
750
500
3827.20
3762.26
3339.09
2930.30
2878.33
1883.27
1862.87
1835.13
1733.49
1634.62
1561.37
1512.81
1456.72
1439.13
1373.60
1357.87
1270.14
1177.43
1064.23
943.54
859.18
816.90
768.20
664.33
600.07
573.00
559.02
504.98

FIG. 2

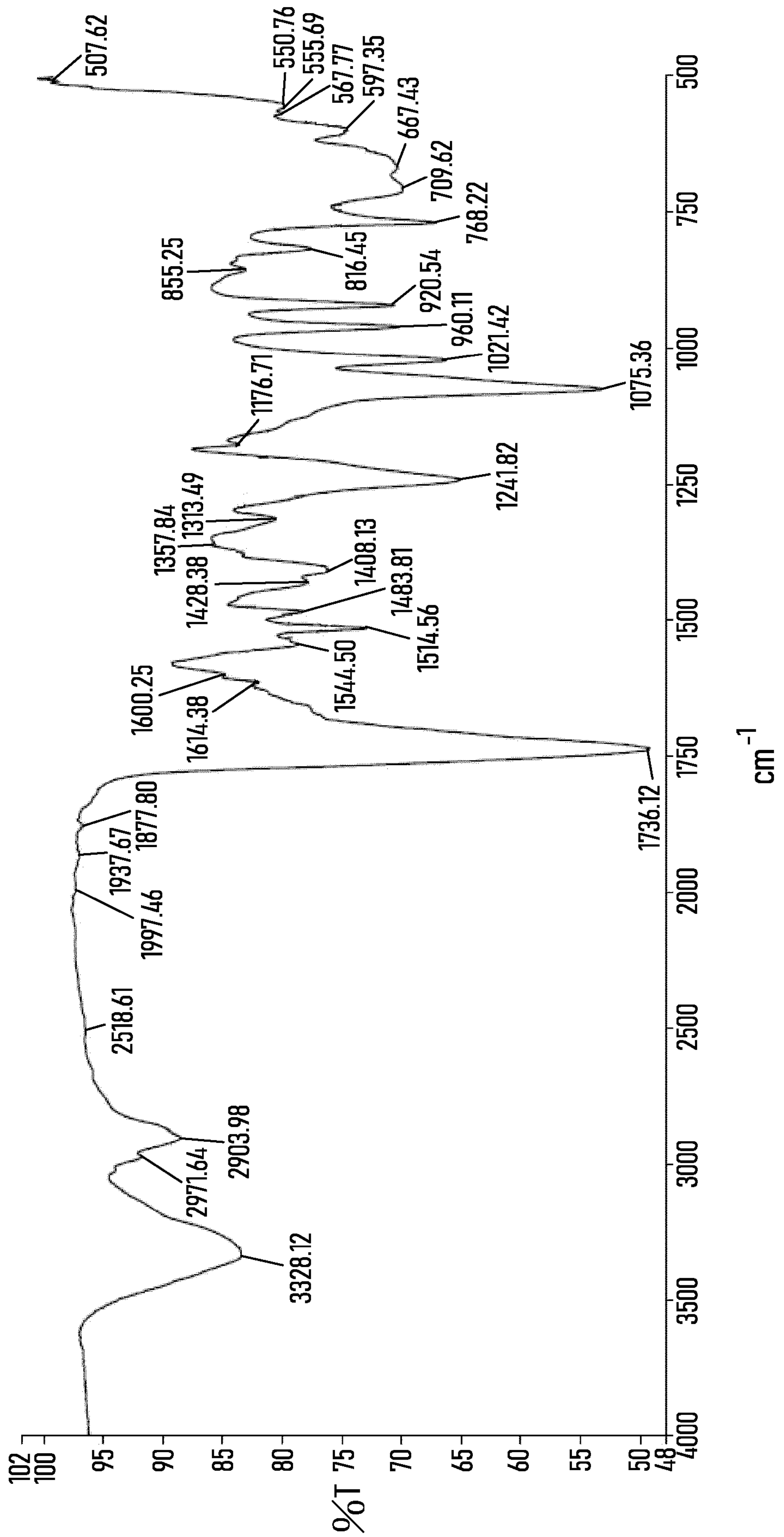

%T
102
100
95
90
85
80
75
70
65
60
55
50
48
4000
3500
3000
2500
2000
1750
1500
1250
1000
750
500
cm-1
3328.12
2971.64
2903.98
2518.61
1997.46
1937.67
1877.80
1736.12
1614.38
1600.25
1544.50
1514.56
1483.81
1428.38
1408.13
1357.84
1313.49
1241.82
1176.71
1075.36
1021.42
960.11
920.54
855.25
816.45
768.22
709.62
667.43
597.35
567.77
555.69
550.76
507.62


FIG. 3

## INTERNATIONAL SEARCH REPORT

International application No.
**PCT/CN2023/131434**

### A. CLASSIFICATION OF SUBJECT MATTER

C08J11/28(2006.01)i；C08J11/22(2006.01)i；C08L75/04(2006.01)i

According to International Patent Classification (IPC) or to both national classification and IPC

### B. FIELDS SEARCHED

Minimum documentation searched (classification system followed by classification symbols)

IPC:C08J,C08L

Documentation searched other than minimum documentation to the extent that such documents are included in the fields searched

Electronic data base consulted during the international search (name of data base and, where practicable, search terms used)

CNTXT, DWPI, ENTXT, ENTXTC, WPABS, WPABSC, CJFD, CNKI, STN, Elsevier Science, ACS, Springer Link: 上纬创新育成, 三井武田, 武田, 陈耀煌, 汪孟纬, 聚氨酯, 废料, 回收, 多元醇, 碳酸乙烯酯, 碳酸丙烯酯, 碳酸酯, 恶唑烷酮, 唑酮, 恶唑酮, #烷酮, 噁#烷酮, 噁#酮, 胺, 氨, SWANCOR, Chen Yaohuang, Wang Mengwei, polyurethane, PU, waste, scrap, recycl+, polyol+, Ethylene carbonate, propylene carbonate, carbonate, +oxazolidone, +oxazolidine, +oxazole, +amino+, +amine+

### C. DOCUMENTS CONSIDERED TO BE RELEVANT

| Category* | Citation of document, with indication, where appropriate, of the relevant passages | Relevant to claim No. |
|---|---|---|
| X | JP 2002105194 A (MITSUI TAKEDA CHEMICALS, INC.) 10 April 2002 (2002-04-10) description, paragraphs 9-10, 17, and 35, decomposition example 1, and embodiment 3 | 1-3, 6-14 |
| A | JP 2002105194 A (MITSUI TAKEDA CHEMICALS, INC.) 10 April 2002 (2002-04-10) entire document | 4-5 |
| A | CN 103374145 A (FOSHAN GAOMING DISTRICT YESHENG POLYURETHANE CO., LTD.) 30 October 2013 (2013-10-30) entire document | 1-14 |
| A | DE 4427250 A1 (BAYER AG) 08 February 1996 (1996-02-08) entire document | 1-14 |
| A | JP 2000247917 A (TAKEDA CHEMICAL INDUSTRIES, LTD.) 12 September 2000 (2000-09-12) entire document | 1-14 |
| A | JP S5586814 A (BAYER AG) 01 July 1980 (1980-07-01) entire document | 1-14 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **25 July 2024** | **30 July 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2023/131434**

**C. DOCUMENTS CONSIDERED TO BE RELEVANT**

| Category* | Citation of document, with indication, where appropriate, of the relevant passages | Relevant to claim No. |
|---|---|---|
| A | US 5763692 A (BASF CORPORATION) 09 June 1998 (1998-06-09) entire document | 1-14 |
| A | JP 2001348457 A (MITSUI TAKEDA CHEMICALS, INC.) 18 December 2001 (2001-12-18) entire document | 1-14 |

Form PCT/ISA/210 (second sheet) (July 2022)

**INTERNATIONAL SEARCH REPORT**
**Information on patent family members**

International application No.
**PCT/CN2023/131434**

| Patent document cited in search report | | | Publication date (day/month/year) | Patent family member(s) | | | Publication date (day/month/year) |
|---|---|---|---|---|---|---|---|
| JP | 2002105194 | A | 10 April 2002 | None | | | |
| CN | 103374145 | A | 30 October 2013 | CN | 103374145 | B | 20 April 2016 |
| DE | 4427250 | A1 | 08 February 1996 | DE | 4427250 | B4 | 16 September 2004 |
| JP | 2000247917 | A | 12 September 2000 | JP | 4501013 | B2 | 14 July 2010 |
| JP | S5586814 | A | 01 July 1980 | DE | 2964320 | D1 | 20 January 1983 |
| | | | | EP | 0013350 | A1 | 23 July 1980 |
| | | | | EP | 0013350 | B1 | 15 December 1982 |
| | | | | DE | 2854940 | A1 | 10 July 1980 |
| | | | | US | 4282367 | A | 04 August 1981 |
| US | 5763692 | A | 09 June 1998 | CA | 2214180 | A1 | 28 April 1998 |
| | | | | EP | 0838492 | A2 | 29 April 1998 |
| | | | | EP | 0838492 | A3 | 01 July 1998 |
| | | | | KR | 19980033202 | A | 25 July 1998 |
| | | | | JP | H10168225 | A | 23 June 1998 |
| JP | 2001348457 | A | 18 December 2001 | CN | 1321700 | A | 14 November 2001 |
| | | | | KR | 20010095083 | A | 03 November 2001 |
| | | | | US | 2001027246 | A | 04 November 2001 |
| | | | | US | 6515036 | B2 | 04 February 2003 |
| | | | | EP | 1142945 | A2 | 10 October 2001 |
| | | | | EP | 1142945 | A3 | 05 June 2002 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 1290908 C